Europäisches Patentamt

(19) European Patent Office

Office européen des brevets

(11) Publication number: **0 288 285 B1**

(12) **EUROPEAN PATENT SPECIFICATION**

(45) Date of publication of patent specification : 13.11.91 Bulletin 91/46

(51) Int. Cl.⁵ : **A41F 1/00, A44B 21/00**

(21) Application number : **88303609.7**

(22) Date of filing : **21.04.88**

(54) **Diaper or babies' napkin fastener.**

(30) Priority : **22.04.87 ZA 872817**

(43) Date of publication of application : **26.10.88 Bulletin 88/43**

(45) Publication of the grant of the patent : **13.11.91 Bulletin 91/46**

(84) Designated Contracting States : **AT BE CH DE ES FR GB IT LI NL SE**

(56) References cited :
**CH-A- 607 049**
**FR-A- 621 124**
**US-A- 2 912 735**
**US-A- 3 051 178**
**US-A- 3 259 916**

(73) Proprietor : **SNAPPI HOLDINGS (PROPRIETARY) LIMITED**
**410 Standard Plaza, 440 Hilda Street, Hatfield**
**Pretoria, Transvaal Province (ZA)**

(72) Inventor : **Visser, Hendrik Schalk**
**160, Stormvoel Street**
**East Lynne, Pretoria, Transvaal Province (ZA)**

(74) Representative : **Walter, Douglas Ernest et al**
**HASELTINE LAKE & CO. 28, Southampton**
**Buildings**
**London WC2A 1AT (GB)**

## Description

This invention relates to a diaper or babies' napkin fastener particularly adapted for use in conjunction with diapers (as babies' napkins will herein be termed) which are made of cloth or woven fabric, as opposed to disposable paper products. The fastener of this invention is, however, not confined in application to such cloth or woven fabric diapers.

A diaper fastener is known from US-A-3051178.

Diapers can basically be divided into two categories, namely disposable diapers made predominantly of paper products, and which are generally fastened by means of fastening arrangements which include adhesive, in particular pressure sensitive adhesives whereby two overlapping parts of a diaper can be secured together optionally by means of a strap, and a second category comprising cloth or woven fabric diapers, particularly those made of towelling material, and which are washable for re-use.

The latter types of diaper are traditionally fastened using one or two safety pins to secure overlapping portions of the cloth or fabric together. The use of such safety pins is not only dangerous, both to the baby and person installing the safety pins in an operative position, but also is tedious.

It is the, object of this invention to provide a fastener for use in conjunction with such cloth or fabric diapers which can more easily be rendered operative, and which may provide added comfort to a baby wearing a diaper fastened with such fastener which also is less dangerous or likely to cause injury either the baby or person fitting the diaper.

In accordance with this invention there is provided a fastener comprising a substantially planar base member having a central zone from which at least 3 arms extend, at least one of such arms being elastically extensible, and a hook or tooth formation associated with each end region of each arm, such hook or tooth formation being operative to engage the fabric of a diaper to anchor the associated arm end against movement thereof relative to the diaper in the direction in which the arm extends.

Further features of the invention provide for 3 arms to radiate from the central zone ; for the central zone and 3 arms to be integral with each other and to be made of elastomeric material ; for the entire base member consisting of the central zone and arms to be made of injection moulded elastomeric plastics material ; for each elastically extensible arm to carry a separately manufactured and substantially rigid unit defining the hook or tooth formations ; and for such separately manufactured units to each have at least one headed formation passing through a hole in the associated arm to retain the unit on such associated arm.

Still further features of the invention provide for each hook or tooth formation to comprise a series of teeth directed inwardly and generally in the direction of the length of the associated arm ; and for two of said arms to be arranged in a substantially co-linear relationship but extending in opposite directions from the central zone in which case the other arm or arms extend outwardly at roughly right angles thereto.

In order that the invention may be more fully understood one embodiment thereof will now be described with reference to the accompanying drawings.

In the drawings :

Figure 1 — is a perspective view illustrating a diaper on a baby and held in position by means of a fastener according to the invention ;

Figure 2 — is a plan view of a base member according to the invention without hook or tooth units associated therewith ;

Figure 3 — is a partly sectioned elevation thereof illustrating one half of the base member in cross-section and one tooth defining unit in exploded relationship relative thereto ;

Figure 4 — is an end view of the tooth defining unit of Figure 3 taken in direction of arrow "A" in Figure 3, and,

Figure 5 — is an inverted plan view of the tooth defining unit in Figures 3 and 4.

In the embodiment of the invention illustrated in the drawings a fastener, generally indicated by numeral 1, is composed of a base 2 made of injection moulded, elastomeric plastics material.

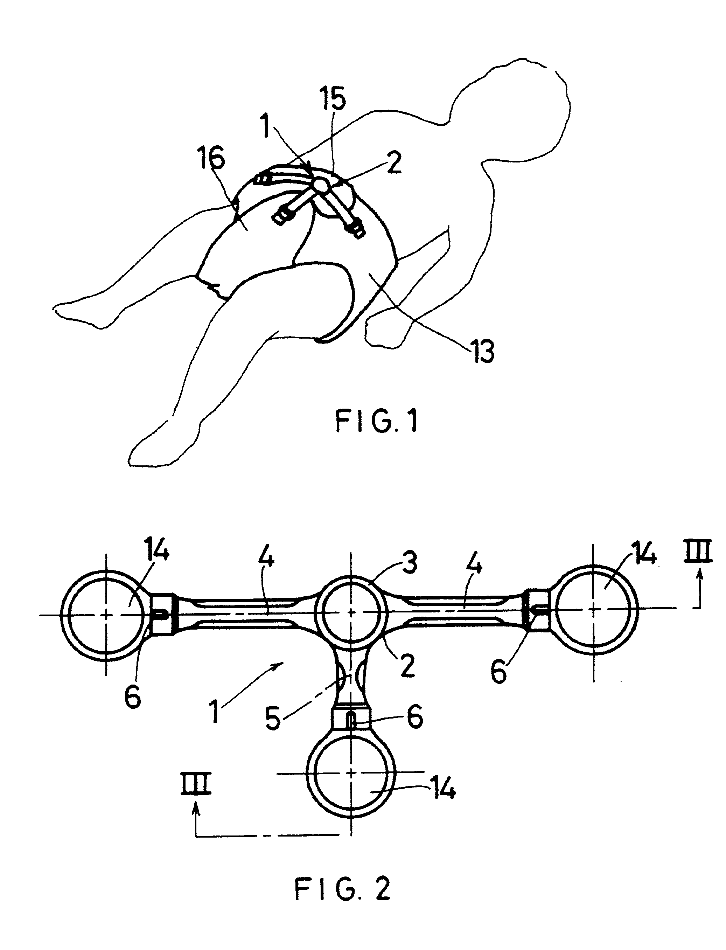

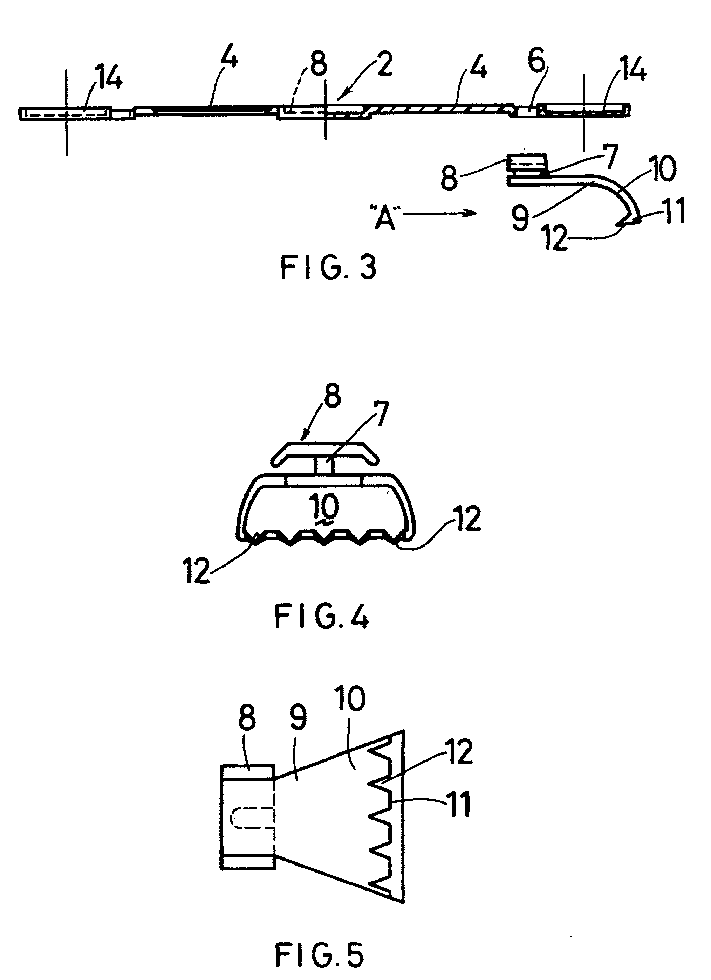

The base member, as shown most clearly in Figures 2 and 3, is moulded as one integral unit and comprises a central zone 3 from which radiate 3 arms, two 4 of which extend collinearly in diametrically opposite directions and a shorter one 5 of which extends at right angles thereto. Each of the arms is, accordingly, elastomerically extensible.

Towards the end of each arm is an elongated aperture 6 passing therethrough and adapted to accommodate the stem 7 of a headed formation 8 associated with a tooth defining unit 9 injection moulded from relatively substantially rigid plastics material. Although only one such unit is illustrated in association with the base in Figure 3, each of the arms has one of such units.

2

Each of the hook defining members 9 extends, in the operative position, in the general direction of the arm with which it is associated and away from the central zone 3 and the hook defining member thereafter passes through an arcuate zone 10 to terminate in a free edge 11 carrying inwardly directed teeth 12.

The direction in which the teeth 12 extend is somewhat away from the plane of the base but generally in a direction towards the central zone. These teeth are adapted to catch and engage in the fibres of a cloth or fabric diaper, indicated by numeral 13 in figure 1.

Moulded integrally with each of the arms is an end tab 14 which, in the operative position, lies above the associated tooth defining unit 9 and which defines a finger grip to assist in extension of the associated arm simply by pulling on the tab.

In use, a babies diaper 13, of the conventional towelling material for example, is passed around the torso of a baby and a central zone is passed upwardly between the legs in known manner.

The two overlapping portions 15 defining the ends of the portion of the diaper passing around the torso, each have one of the tooth defining units engaged therewith in succession with the collinear arms 4 interconnecting same being extended suitably to provide a desired close fit of the diaper around the torso.

The shorter arm 5 is then extended and its associated tooth defining unit 9 engaged with the portion 16 of the diaper passing upwardly between the legs of the baby.

It will be understood that the above described fastener will hold the diaper firmly in position and, due to its elasticity will indeed allow for movement and, it is considered, greater comfort to the baby. It will be seen that installation of the fastener provided by this invention is both swift and simple as well as being safe and not involving any danger of either the baby or the person installing the diaper from being pricked accidentally by a safety pin in all too well-known fashion.

In a variation of the present invention the single shorter arm could be replaced by two or more spaced arms for engaging the portion of a diaper passing between the legs of a baby.

## Claims

1. A fastener (1) comprising a substantially planar (2) base member having a central zone (3) from which at least three arms (4, 5) extend, and a hook or tooth formation (9) associated with each end region (14) of each arm, such hook or tooth formation being operable to engage the fabric of a diaper to anchor the associated arm end against movement thereof relative to the diaper in the direction in which the arm extends, characterised in that at least one of said arms is elastically extensible.

2. A fastener as claimed in claim 1 in which the central zone and arms are integral with each other and are all made of elastomeric material.

3. A fastener as claimed in either of claims 1 or 2 in which the base member is made of injection moulded elastomeric plastics material.

4. A fastener as claimed in any one of claims 1 to 3 in which three arms (4, 5) radiate from a central zone (3), two of such arms (4) being substantially collinear.

5. A fastener as claimed in any one of the preceding claims in which each elastically extensible arm carries a separately manufactured, substantially rigid unit (9) defining the hook or tooth formations.

6. A fastener as claimed in claim 5 in which the separately manufactured units (9) each have at least one headed formation (7, 8) passing through a hole (6) in the associated arm to retain the unit on said associated arm.

7. A fastener as claimed in any one of the preceding claims in which the free ends of the arm are provided with finger grips (14) whereby they may be pulled.

8. A fastener as claimed in any one of the preceding claims in which each hook or tooth formation (11) comprises a series of teeth (12) directed inwardly and generally in the direction of the length of the associated arm.

9. A fastener as claimed in any one of the preceding claims in which two of said arms (4) are substantially collinear and extend in opposite directions from the central zone, and the other arm (5) extends outwardly at substantially right angles thereto.

## Patentansprüche

1. Befestiger (1) aufweisend ein im wesentlichen ebenes (2) Grundelement mit einem Mittelbereich (3), von dem sich mindestens drei Arme (4, 5) erstrecken, und eine Haken- oder Zahnbildung (9) für jeden Endbereich (14) jedes Arms, wobei die Haken- oder Zahnbildung in Eingriff mit dem Gewebe einer Windel gebracht

3

werden kann, um das zugehörige Armende gegen Bewegung in Erstreckungsrichtung des Arms relativ zur Windel zu verankern, **dadurch gekennzeichnet**, daß einer der Arme elastisch verlängerbar ist.

2. Befestiger nach Anspruch 1, bei dem der Mittelbereich und die Arme einstückig miteinander ausgebildet und alle aus elastomerem Material hergestellt sind.

3. Befestiger nach Anspruch 1 oder 2, bei dem das Grundelement aus spritzgußgeformtem elastischem Kunststoffmaterial hergestellt ist.

4. Befestiger nach einem der Ansprüche 1 bis 3, bei dem sich die drei Arme (4, 5) von einem Mittelbereich (3) erstrecken, wobei zwei der Arme (4) im wesentlichen kolinear verlaufen.

5. Befestiger nach einem der vorhergehenden Ansprüche, bei dem jeder elastisch verlängerbare Arm eine gesondert gefertigte, im wesentlichen starre Einheit (9) trägt, die die Haken- oder Zahnbildung darstellt.

6. Befestiger nach Anspruch 5, bei dem die getrennt hergestellten Einheiten (9) jede mindestens eine Kopfbildung (7, 8) haben, die sich durch ein Loch (6) im zugehörigen Arm erstreckt, um die Einheit am Arm zu halten.

7. Befestiger nach einem der vorhergehenden Ansprüche, bei dem die freien Enden der Arme mit Fingerflächen (14) versehen sind, so daß an ihnen gezogen werden kann.

8. Befestiger nach einem der vorhergehenden Ansprüche, bei dem jede Haken- oder Zahnbildung (11) eine Anzahl von Zähnen (12) aufweist, die nach innen und im allgemeinen in Richtung der Längserstreckung des zugehörigen Arms gerichtet sind.

9. Befestiger nach einem der vorhergehenden Ansprüche, bei dem zwei der Arme (4) im wesentlichen kolinear verlaufen und sich in entgegengesetzten Richtungen vom Mittelbereich erstrecken und bei dem der andere Arm (5) sich im wesentlichen rechtwinklig dazu nach außen erstreckt.


**Revendications**

1. Dispositif de fixation (1) comportant un élément de base sensiblement plan (2) pourvu d'une zone centrale (3) à partir de laquelle s'étendent au moins trois bras (4, 5), et une structure formant crochet ou denture (9) associée à chaque partie d'extrémité (14) de chacun des bras, structure formant crochet ou denture qui est apte à opérer pour venir en prise avec le tissu d'une couche, afin d'ancrer l'extrémité de bras associée à l'encontre d'un déplacement de celle-ci par rapport à la couche, dans la direction dans laquelle s'étend le bras, caractérisé en ce que l'un au moins desdits bras est extensible élastiquement.

2. Dispositif de fixation selon la revendication 1, dans lequel la zone centrale et les bras sont solidaires les uns des autres et tous faits d'un matériau en élastomère.

3. Dispositif de fixation selon la revendication 1 ou 2, dans lequel l'élément de base est fait d'une matière plastique en élastomère moulée par injection.

4. Dispositif de fixation selon l'une quelconque des revendications 1 à 3, dans lequel trois bras (4, 5) rayonnent à partir d'une zone centrale (3), deux (4) de ces bras étant sensiblement en alignement l'un avec l'autre.

5. Dispositif de fixation selon l'une quelconque des revendications précédentes, dans lequel chaque bras extensible élastiquement porte un élément sensiblement rigide, fabriqué séparément (9) qui définit les structures formant crochet ou denture.

6. Dispositif de fixation selon la revendication 5, dans lequel les éléments fabriqués séparément (9) comportent respectivement au moins une structure (7, 8) pourvue d'une tête, passant par un trou (6) prévu dans le bras associé, afin de retenir l'élément sur ledit bras associé.

7. Dispositif de fixation selon l'une quelconque des revendications précédentes, dans lequel les extrémités libres des bras sont pourvue d'organes de préhension (14), à l'aide desquels elles peuvent être tirées.

8. Dispositif de fixation selon l'une quelconque des revendications précédentes, dans lequel chaque structure formant crochet ou denture (11) comporte une série de dents (12) dirigées vers l'intérieur et, d'une manière générale, dans le sens de la longueur du bras associé.

9. Dispositif de fixation selon l'une quelconque des revendications précédentes, dans lequel deux (4) desdits bras sont sensiblement en alignement l'un avec l'autre et s'étendent dans des directions opposées à partir de la zone centrale, tandis que l'autre bras (5) s'étend vers l'extérieur, sensiblement à angle droit par rapport à eux.

FIG.1

FIG. 2

FIG. 3

FIG. 4

FIG. 5